(19) Europäisches Patentamt / European Patent Office / Office européen des brevets

(11) **EP 3 443 891 B1**

(12) **FASCICULE DE BREVET EUROPEEN**

(45) Date de publication et mention de la délivrance du brevet:
**16.09.2020 Bulletin 2020/38**

(21) Numéro de dépôt: **18198363.6**

(22) Date de dépôt: **07.11.2013**

(51) Int Cl.:
*A61B 5/00* (2006.01)     *A61B 8/08* (2006.01)
*A61B 8/00* (2006.01)     *A61C 19/04* (2006.01)
*B06B 1/06* (2006.01)

(54) **SONDE ET DISPOSITIF ULTRASONORE D'IMAGERIE 3D DE LA MACHOIRE**

ULTRASCHALLSONDE UND VORRICHTUNG FÜR 3D-BILDGEBUNG DES KIEFERS

ULTRASOUND PROBE AND DEVICE FOR 3D IMAGING OF THE JAW

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.11.2012 FR 1260617**

(43) Date de publication de la demande:
**20.02.2019 Bulletin 2019/08**

(62) Numéro(s) de document de la (des) demande(s) initiale(s) en application de l'article 76 CBE:
**13798953.9 / 2 916 718**

(73) Titulaire: **TROPHY SAS**
**77435 Marne La Vallee Cedex 2 (FR)**

(72) Inventeurs:
• **GERBAULET, Jean-Pierre**
**77435 Marne La Vallée Cedex 2 (FR)**
• **GREGOIRE, Jean-Marc**
**37390 Mettray (FR)**
• **LEVASSORT, Franck**
**37550 Saint-Avertin (FR)**

(74) Mandataire: **Klang, Alexander H.**
**Wagner & Geyer Partnerschaft mbB**
**Patent- und Rechtsanwälte**
**Gewürzmühlstrasse 5**
**80538 München (DE)**

(56) Documents cités:
**WO-A1-95/04506**     **WO-A1-2008/137030**
**US-A1- 2012 244 489**     **US-B1- 6 638 219**

• **M.C.D.N.J.M HUYSMANS ET AL: "Ultrasonic measurement of enamel thickness: a tool for monitoring dental erosion?", JOURNAL OF DENTISTRY, vol. 28, no. 3, 1 mars 2000 (2000-03-01), pages 187-191, XP055069078, ISSN: 0300-5712, DOI: 10.1016/S0300-5712(99)00063-9**
• **ASHRAF F. EL-SHERIF ET AL: "Laser-induced photothermal technique used for detection of caries in human tooth", PROCEEDINGS OF SPIE, vol. 6843, 7 février 2008 (2008-02-07), page 68430B, XP055517183, 1000 20th St. Bellingham WA 98225-6705 USA ISSN: 0277-786X, DOI: 10.1117/12.759157 ISBN: 978-1-5106-2011-7**

EP 3 443 891 B1

## Description

## Domaine technique

La présente invention concerne une sonde ultrasonore d'imagerie dentaire. Elle concerne aussi un dispositif d'imagerie dentaire comprenant une telle sonde, et un procédé d'imagerie dentaire utilisant une telle sonde.

Un tel dispositif permet à un utilisateur de mesurer le profil de l'os de soutien dentaire d'un individu, sa qualité osseuse et les structures incluses.

## Etat de la technique antérieure

L'allongement de la vie et le souci croissant de procurer aux seniors une vie active et confortable participent à l'intérêt suscité par l'implantologie dentaire. Plus personne aujourd'hui ne souhaite avoir dans sa bouche un « appareil », dentier, râtelier ou équivalent, dont la fixation est incertaine, l'hygiène délicate et la durée de vie limitée, pour un coût néanmoins élevé. La solution alternative qui se développe partout dans le monde est la pose de prothèses dentaires individuelles fixées sur des racines artificielles : les implants.

La pose d'implants dentaires constitue une opération chirurgicale délicate, qui nécessite une grande rigueur de la part du praticien qui l'exécute.

Avant la pose, le praticien ouvre généralement la gencive au niveau de l'emplacement du ou des futurs implants afin de dégager l'os. A l'aide d'un ou de plusieurs forets, il perce dans l'os un puits d'insertion, dont le diamètre est légèrement inférieur à celui de l'implant qu'il est destiné à accueillir. L'implant, généralement constitué de titane, est ensuite inséré dans le puits, puis la gencive est refermée. S'en suit une phase passive dite de « mise en nourrice », durant laquelle s'opère la cicatrisation de l'os autour de l'implant. Cette phase de cicatrisation, également appelée « ostéointégration », peut durer de plusieurs semaines à plusieurs mois. Elle est suivie du vissage de la couronne sur l'implant.

La définition de l'axe de perçage, en vue de l'obtention d'une orientation finale précise de l'implant, et la détermination du diamètre et de la profondeur du puits d'insertion, constituent des difficultés majeures de la pose d'implants dentaires. Ainsi, il arrive parfois que la paroi du sinus soit traversée ou que le nerf dentaire soit touché par le foret de perçage lorsque celui-ci est orienté de manière inappropriée ou qu'il va trop loin dans l'os de la mâchoire.

De ce fait, la pose correcte des implants a été longtemps réservée à une minorité de praticiens réputés pour leur habileté, bien qu'opérant de façon empirique, et dont les tarifs étaient généralement élevés.

Depuis les années 90, des techniques ont été développées pour permettre à la plupart des dentistes de poser des implants tout en contrôlant les risques. On les regroupe sous le nom d'implantologie assistée par ordinateur. En voici le principe.

A partir d'images 3D donnant des informations sur la dentition du patient et les structures sous-jacentes (os, nerf, sinus), l'implantologiste peut planifier et simuler l'intervention chirurgicale grâce à un logiciel informatique dans lequel sont stockées des images 3D des implants et piliers prothétiques utilisés. Il en résulte une planification réaliste, précise et fiable, incluant le nombre d'implants requis, leur taille et leur position, ainsi que la sélection de piliers prothétiques appropriés.

Le dentiste réplique ensuite cette simulation lors de la chirurgie, par exemple à l'aide d'un moulage en résine de la mâchoire du patient dans lequel des guides de perçage sont incorporés, conformément à la simulation effectuée. Cet appareil à usage unique, appelé « guide chirurgical », est précisément adapté au cas traité. Des forets à butée limitant la profondeur du perçage complètent le dispositif.

Il est possible d'obtenir un résultat similaire à l'aide d'un procédé dit de « navigation », dans lequel la main du praticien est guidée avec précision par un système optique, informatique et éventuellement robotique.

## Coût de l'implantoloagie assistée par informatique

Jusqu'à une époque récente, le coût élevé par implant posé empêchait la prise en charge de cette prothèse par l'assurance maladie et les mutuelles, ce qui rendait l'implantologie dentaire inaccessible à la majorité des patients. En permettant la généralisation de l'offre en toute sécurité, l'implantologie guidée par informatique devrait modifier cette situation, à la condition que le prix par implant posé diminue de façon substantielle.

Si on peut espérer que la concurrence accrue fera baisser les prix des matériels et les tarifs des praticiens, il n'en est pas de même pour l'imagerie 3D, indispensable à cette technique opératoire, qui utilise actuellement des scanners à Rayons X : le « spiral à barrettes » ou CT scanner, et le « cone beam » ou CBCT.

A titre d'exemple, en 2011, les images scanner d'un centre de radiologie coûtaient en France, de 150 à 450 € suivant les matériels et suivant que l'image représentait une partie de la mâchoire ou la totalité.

Aujourd'hui, malgré une forte percée des CBCTs dans les cabinets d'une certaine importance, l'imagerie 3D pourrait être un obstacle à la démocratisation de l'implantologie guidée, car son coût reste élevé (de l'ordre de 100.000 euros à l'achat pour un CBCT).

De plus, pour les cabinets non équipés de CBCT, la sous-traitance de l'examen rallonge les délais de traitement, parfois d'une façon excessive dans les régions où les scanners sont peu nombreux.

Dans les pays moins développés, la barrière à l'entrée constituée par l'imagerie 3D est d'autant plus élevée que les scanners y sont plus rares et que le coût des images y est proportionnellement plus élevé.

D'un point de vue économique et pratique, l'imagerie 3D est donc l'un des maillons faibles de la chaîne technique nécessaire à une large diffusion de l'implantologie guidée. De ce fait, un appareil bon marché, installé au cabinet, présenterait un avantage certain pour l'économie de santé publique.

## L'irradiation due à l'imagerie 3D à rayons X (RX)

Il convient par ailleurs de soulever un autre problème de l'imagerie 3D utilisant les rayons X : la dose d'irradiation qu'elle fait subir au patient. Pour reconstruire une image 3D, un scanner nécessite un nombre important de clichés 2D. De ce fait, alors que l'irradiation correspondant à une radiographie panoramique classique est de l'ordre de 15 mgy (milligray), celle d'un scanner dentaire est comprise entre 200 et 400 mgy !

Des études réalisées en 2007 aux Etats-Unis (Amy Berrington de Gonzalez et al, Rebecca Smith-Bindman et al) montrent que le risque de tumeur imputable à des examens utilisant des rayons X (cancers radio-induits) est important, surtout si les examens sont répétitifs. Or ces examens sont en croissance exponentielle depuis quelques années.

D'après Longstreth et al, cinq bilans radiographiques dentaires augmentent le risque de voir se développer un méningiome intra-cranien.

D'après cinq études épidémiologiques de Preston-Martin et White, des tumeurs des glandes salivaires et crâniennes sont associées à des rayonnements cumulatifs de diagnostics dentaires.

D'après Hallquist et al, les rayonnements dus aux examens de la face et du crâne, en particulier les radiographies dentaires, induisent un risque d'augmentation des cancers de la tyroïde.

Une étude de Memon et al montre que l'apparition d'un cancer de la tyroïde est directement liée à la prise de radiographies dentaires.

Depuis 2005, la pénétration importante réalisée par les CBCTs dans les cabinets dentaires (il existe aujourd'hui 25 fabricants), et principalement dans les cabinets d'implantologie, du fait de l'autonomie procurée au praticien, n'a pas résolu ce problème comme on aurait pu l'espérer. Une série d'articles publiés dans le New York Times en 2010 dénonce en effet l'utilisation excessive qui est faite des CBCTs dans les cabinets dentaires, en implantologie, en orthodontie et même en pédiatrie.

Même si les doses sont moindres que celles émises lors d'examens similaires avec des scanners traditionnels (spiral à barrettes), le Dr. John Ludlow de l'Université de l'Ecole Dentaire de Caroline du Nord a montré que les CBCT délivrent de 4 à 67 fois plus de radiations que les radiographies panoramiques digitales, en cohérence avec les chiffres cités plus haut. Les autorités sanitaires de plusieurs pays recommandent donc de limiter autant que possible les doses subies par les patients lorsque l'on utilise les rayons X, sans pour autant en recommander l'abandon. En effet, le rejet de l'imagerie diagnostique n'est plus concevable à notre époque.

## Utilisation des ultrasons

En 1999 Muller a présenté un article qui décrivait la mesure de l'épaisseur de la muqueuse masticatoire avec un appareil commercial spécifique (« Ultrasonic détermination of thickness of masticatory mucosa », Oral Surgery Oral Medicine Oral Pathology, vol.88 n°2, August 1999). Cet appareil de marque KRUPP et nommé SDM est constitué d'une petite sonde stylo avec sur son embout coudé, pour faciliter son placement sur la gencive, un transducteur avec un diamètre actif 4 mm. Il indique que cet appareil permet de mesurer l'épaisseur de la gencive en un point fixé manuellement par le praticien. Mais ce type d'appareil n'est pas satisfaisant pour un praticien désirant planifier une chirurgie avec suffisamment de précision.

En 2001, la société israélienne Imadent a déposé un brevet (US 7 285 093 B2) sur un système s'apparentant à de la tomographie par ultrasons. Mais cette technologie n'est pas satisfaisante pour un praticien désirant planifier une chirurgie avec suffisamment de précision. Depuis, le brevet a été abandonné.

Le document US 2012 0 244 489 A1 divulgue une sonde ultrasonore d'examen dentaire comprenant:

des moyens pour émettre des ondes ultrasonores, ces moyens d'émission d'ondes ultrasonores comprenant une matrice ultrasonore souple comprenant:

-- un support souple,
-- plusieurs transducteurs agencés pour fonctionner en émission et en réception et montés sur le support souple et disposés sous forme de matrice,

une armature rigide solidaire et en contact avec la matrice ultrasonore souple, de sorte que chaque transducteur donné soit agencé pour émettre une onde ultrasonore dans une direction opposée à l'armature rigide. Cette sonde forme l'état de la technique le plus proche de l'invention.

Le but de l'invention est de proposer un dispositif peu ou pas irradiant, pouvant être utilisé en tant qu'appareil d'imagerie 3D (par exemple pour fournir à un dentiste les images lui permettant de planifier sa chirurgie), et de préférence avec une résolution suffisante et/ou un coût permettant de pouvoir être installé et exploité de manière intéressante par un praticien dans un cabinet dentaire.

## Exposé de l'invention

Cet objectif est atteint avec une sonde ultrasonore d'imagerie dentaire, comme elle est définie dans la revendication 1, et avec un dispositif d'imagerie dentaire, comme il est définie dans la revendication 10.

De préférence, chaque transducteur « haute fréquence » donné est agencé pour émettre une onde ultrasonore dans une direction perpendiculaire à la surface du support souple portant ce transducteur « haute fréquence » donné.

De préférence, chaque transducteur « haute fréquence » donné est agencé pour émettre une onde ultrasonore dans une direction opposée (et de préférence perpendiculaire) à la surface du support souple portant ce transducteur « haute fréquence » donné.

De préférence, l'armature rigide est solidaire et en contact avec le support souple de la matrice (mais de préférence pas en contact des transducteurs « haute fréquence ») de sorte que le support souple épouse la forme de l'armature rigide.

Les moyens pour émettre des ondes ultrasonores sont en outre de préférence agencés pour émettre des ondes ultrasonores de fréquence inférieure à 4 MHz, la matrice ultrasonore souple comprenant en outre de préférence plusieurs transducteurs « basse fréquence » eux aussi portés par le support souple et agencés pour fonctionner en émission et en réception et agencés pour émettre des ondes ultrasonores de fréquence inférieure à 4 MHz et montés sur le support souple de sorte que chaque transducteur « basse fréquence » donné soit agencé pour émettre une onde ultrasonore dans une direction opposée (et de préférence perpendiculaire) à l'armature rigide, typiquement dans la direction de l'os à imager.

De préférence, chaque transducteur « basse fréquence » donné est agencé pour émettre une onde ultrasonore dans une direction perpendiculaire à la surface du support souple portant ce transducteur « basse fréquence » donné.

De préférence, chaque transducteur « basse fréquence » donné est agencé pour émettre une onde ultrasonore dans une direction opposée (et de préférence perpendiculaire) à la surface du support souple portant ce transducteur « basse fréquence » donné.

De préférence, l'armature rigide est solidaire et en contact avec le support souple de la matrice (mais de préférence pas en contact des transducteurs « basse fréquence ») de sorte que le support souple épouse la forme de l'armature rigide.

Le support souple peut être monté de manière amovible dans l'armature rigide pour une interchangeabilité de l'armature rigide.

L'armature rigide a de préférence une forme courbe (« forme en U ») présentant un côté concave entourant un axe central et un côté convexe, la forme courbe étant délimitée par deux bords latéraux disposés face à face et reliés par un fond, le support souple étant solidaire de l'armature rigide du côté concave de l'armature rigide. Les deux bords latéraux sont de préférence distants, du côté concave, d'une distance comprise soit entre 8mm et 30mm soit entre 4cm et 20cm.

Le fond peut présenter au moins une découpe qui rend sa largeur, mesurée selon une direction parallèle à l'axe central, inférieure à la largeur des bords latéraux.

Chacun parmi les deux bords latéraux et le fond de l'armature rigide sont de préférence solidaires d'une partie de la matrice ultrasonore, partie sur laquelle sont montés des transducteurs (aussi bien « haute fréquence », que « basse fréquence » si la sonde en comprend).

Un transducteur est un dispositif convertissant une grandeur physique en une autre.

Les transducteurs de la sonde selon l'invention sont agencés pour, en cas de « réception » convertir une onde acoustique (ultrasonore) en une autre grandeur physique, et en cas « d'émission » convertir cette autre grandeur physique en onde acoustique (ultrasonore).

Les transducteurs (aussi bien « haute fréquence » que « basse fréquence » si la sonde en comprend) sont de préférence des éléments électro-acoustiques (de préférence de type piézo-électrique) ou autre (par exemple opto-acoustiques) et/ou peuvent éventuellement être entrelacés avec des guides optiques.

Electro-acoustique caractérise tout système ou tout matériau capable de produire une onde acoustique (sonore ou ultrasonore) à partir d'un signal électrique et/ou inversement, tout système et matériau capable de générer un signal électrique à partir d'une onde ou signal acoustique. Les céramiques et films piézoélectriques, les cMUTs sont des exemples de systèmes électroacoustiques.

De même, opto-acoustique caractérise tout système (ou tout matériau) capable de produire une onde acoustique (sonore ou ultrasonore) à partir d'un signal optique (et/ou inversement, tout système et matériau capable de générer un signal optique à partir d'une onde ou signal acoustique).

Les transducteurs (aussi bien « haute fréquence » que « basse fréquence » si la sonde en comprend) sont de préférence disposés en une matrice de transducteurs élémentaires, linéaire à une dimension ou à deux dimensions formant une surface, voire à trois dimensions formant une surface ou un volume.

Le dispositif selon l'invention peut comprendre des moyens de connexion entre la sonde et les moyens de commande, les moyens de connexion pouvant être déconnectés et re-connectés pour débrancher un premier type de sonde des moyens de commande et rebrancher aux moyens de commande un deuxième type de sonde.

Suivant encore un autre aspect de l'invention, il est proposé un premier procédé d'utilisation d'un dispositif selon l'invention.

**Description des figures et modes de réalisation**

D'autres avantages et particularités de l'invention apparaîtront à la lecture de la description détaillée de mises en œuvre et de modes de réalisation nullement limitatifs, et des dessins annexés suivants :

- la figure 1 illustre un dispositif 51 selon l'invention, comprenant une sonde 1 de « type 1 » ou de « type 2 » ;

- la figure 2 illustre une sonde selon l'invention de « type 1 », chevauchant une gencive 7,
- la figure 3 illustre une sonde selon l'invention de « type 1 », chevauchant une gencive 7,
- la figure 4 illustre une sonde selon l'invention de « type 1 », chevauchant une gencive 7,
- la figure 5 illustre une sonde selon l'invention de « type 1 », chevauchant une dent et sa gencive 7,
- la figure 6 illustre une sonde selon l'invention de « type 2 »,
- la figure 7 illustre un module électronique d'un dispositif selon l'invention,
- la figure 8 est une vue de coupe de profil d'une sonde selon l'invention de « type 1 », chevauchant une gencive 7, et utilisée en mode «haute fréquence », (mode « réflectif » pour une définition haute résolution du profil de l'os)
- la figure 9 illustre les différentes ondes et échos lors d'une utilisation d'une sonde selon l'invention en « haute fréquence »,
- la figure 10 est une vue de coupe de profil d'une sonde selon l'invention de « type 1 », chevauchant une gencive 7, et utilisée en mode « basse fréquence », (mode « transmissif » pour une reconstruction de type tomographique)
- la figure 11 est une vue de coupe de dessus d'une sonde selon l'invention de « type 2 », entourant une mâchoire, et utilisée en mode « basse fréquence », (mode « transmissif » pour une reconstruction de type tomographique)
- la figure 12 est une vue d'une sonde selon l'invention chevauchant un fantôme de calibration, et en cours de calibration,
- la figure 13 illustre deux fantômes de calibration, et
- la figure 14 illustre des repères 22 sur la matrice souple 3, 4 d'une sonde selon l'invention.

Ces modes de réalisation n'étant nullement limitatifs, on pourra notamment considérer des variantes de l'invention ne comprenant qu'une sélection de caractéristiques décrites par la suite isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, ou avec seulement une partie des détails structurels si cette partie uniquement est suffisante pour conférer un avantage technique ou à différencier l'invention par rapport à l'état de la technique antérieure.

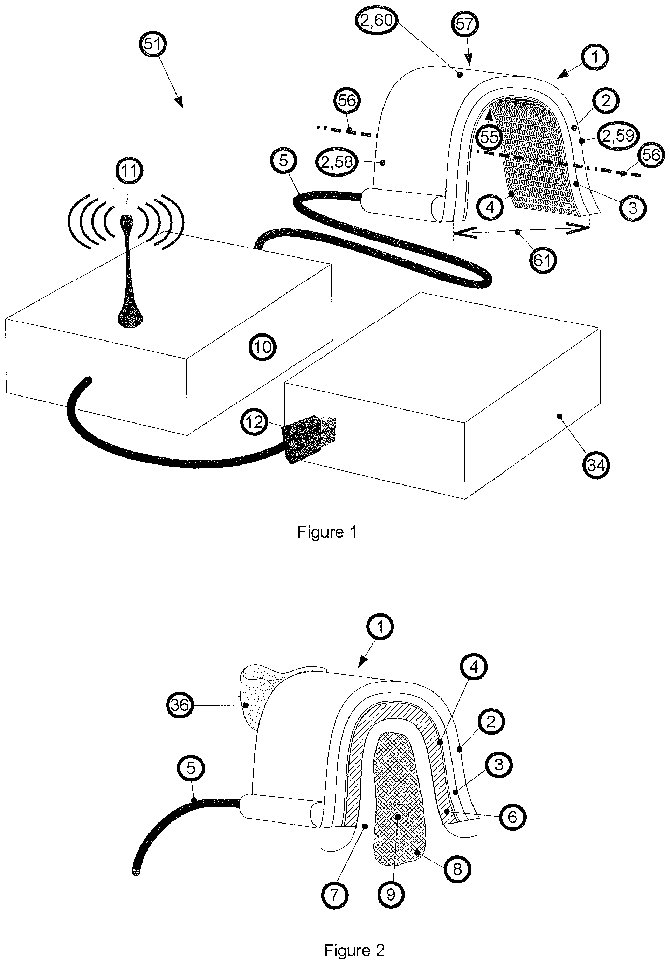

On va tout d'abord décrire, en référence aux figures 1 à 11, un dispositif 51 selon l'invention.

Le dispositif 51 permet d'obtenir une image 3D des os 8 de la mâchoire, maxillaire et/ou mandibule d'une personne, en utilisant des ultrasons pour déterminer les caractéristiques dimensionnelles et les propriétés mécaniques de l'os 8.

## Architecture globale du dispositif 51

Comme illustré sur la figure 1, le dispositif 51 comprend :

- une sonde 1, de préférence interchangeable,
- un module 10 électronique d'alimentation électrique, de commande de la sonde et d'acquisition de données ou signaux en provenance de la sonde 1, et
- un module 34 électronique de traitement des données ou signaux provenant de la sonde.

La sonde 1 est :

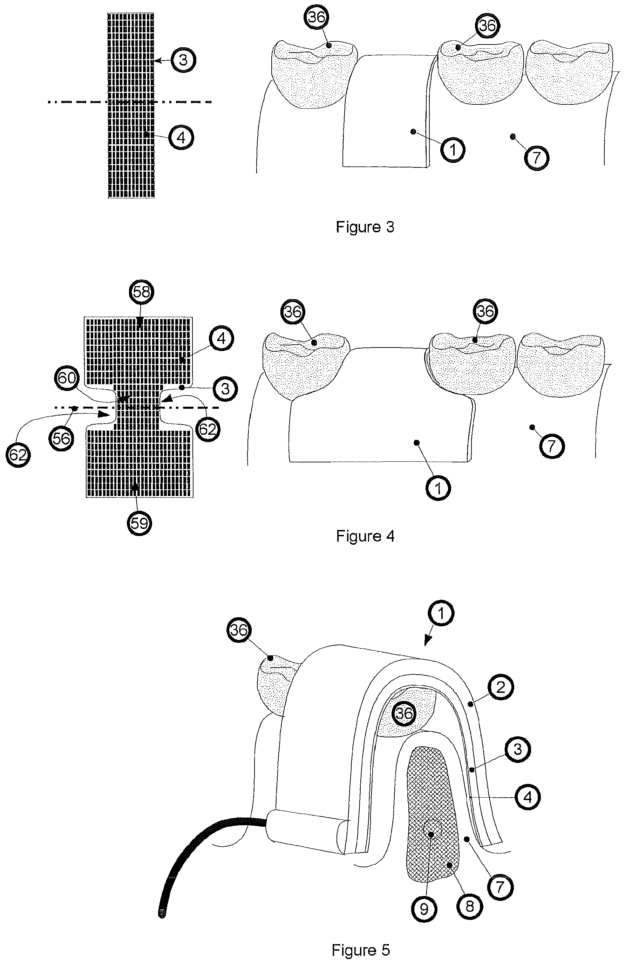

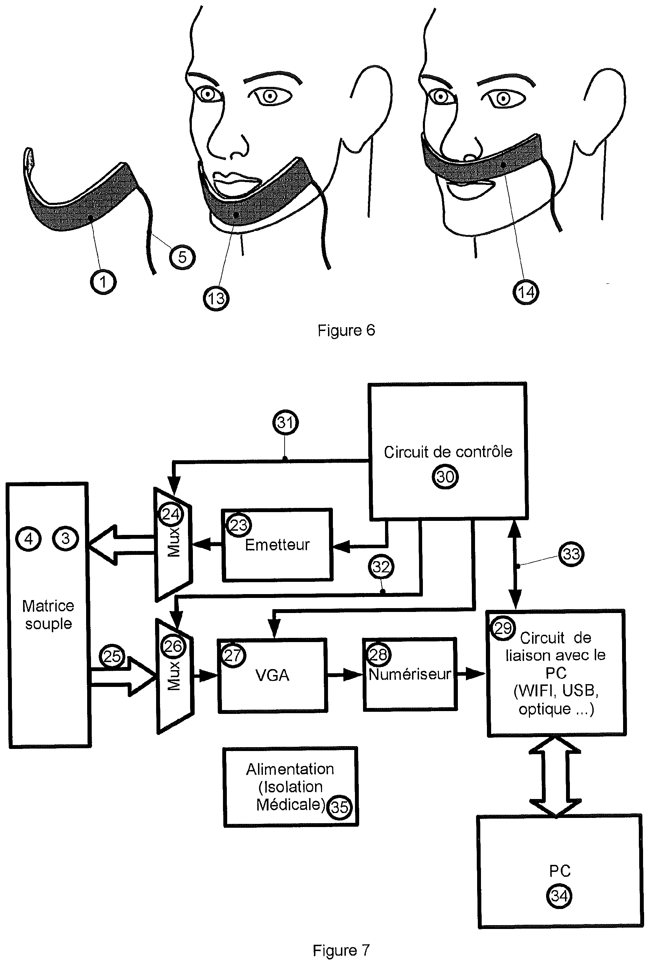

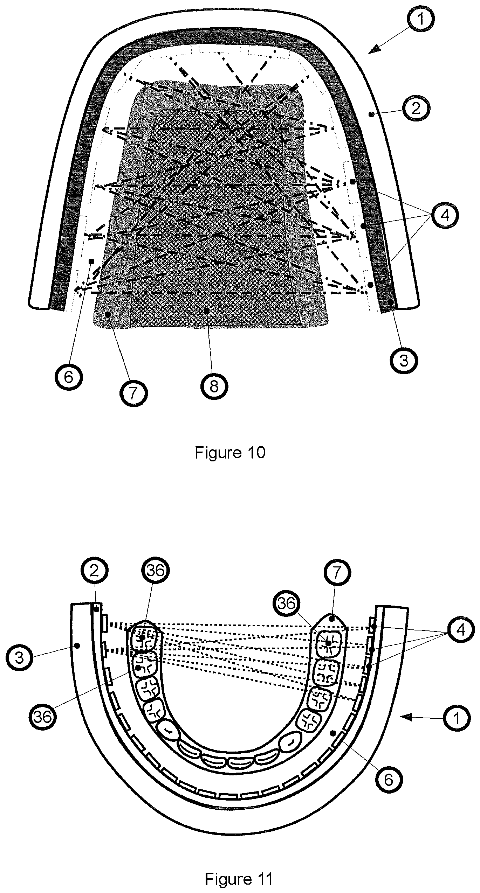

- soit, une sonde 1 spécifique de « type 1 », destinée à être utilisée à l'intérieur d'une bouche (d'un animal, de préférence d'un être humain), qui vient recouvrir une gencive 7 (comme illustré sur la figure 2, 3, 4, 8 ou 10), ou une dent 36 et la gencive 7 (comme illustré sur la figure 5), à travers un matériau de couplage acoustique 6,
- soit une sonde de type 2 (comme illustré sur la figure 6, ou 11) qui englobe tout ou partie de la mâchoire, et est utilisée à l'extérieur 13, 14 de la bouche,

Les signaux obtenus par la sonde 1 lors des mesures sont transmis au module 34, comprenant de préférence un système numérique, par exemple un ordinateur type PC, par des moyens 11 de liaison sans fil hertzienne, WIFI ou autre, ou par des moyens 12 de liaison filaire par exemple par câble, par exemple de type USB, imposant une contrainte supplémentaire d'isolation électrique afin de répondre aux normes de sécurité des appareils électro-médicaux, ou encore de type optique (fibre optique) pour simplifier le respect des règles d'isolation.

Un logiciel spécifique, installé sur le module 34, par exemple sur un ordinateur de type PC, permet de contrôler et de configurer le module électronique 10.

## Structure des sondes ultrasonores

Les sondes de type 1 et 2 sont constituées :

- de moyens pour émettre des ondes ultrasonores, agencés pour émettre des ondes ultrasonores de haute fréquence d'au moins 10 MHz (de préférence au moins 15 MHz, de préférence entre 15MHz et 25MHz), ces moyens d'émission d'ondes ultrasonores comprenant une matrice 3, 4 ultrasonore souple comprenant :

  ∘ plusieurs transducteurs « haute fréquence » 4 agencés pour fonctionner en émission et en réception, et notamment agencés pour émettre des ondes ultrasonores de fréquence d'au moins 10 MHz, voire d'au moins 15 MHZ, de préférence entre 15MHz et 25MHz (c'est-à-dire de préférence que chacun de ces transducteurs « haute fréquence » a une bande passante à -6dB Fsup-Finf , de sa réponse impulsionnelle, centrée sur une fréquence centrale (Finf+Fsup)/2 d'au moins 10 MHz, voir d'au moins 15 MHz, de préférence entre 15MHz et 25MHz),

○ un support souple 3 sur lequel sont montés les transducteurs « haute fréquence » 4 de sorte que chaque transducteur « haute fréquence » donné 4 soit agencé pour émettre une onde ultrasonore dans une direction 16 opposée et perpendiculaire à la surface du support souple 3 portant ce transducteur « haute fréquence » donné 4,

- une armature rigide 2 solidaire de la matrice ultrasonore 3, 4 et en contact du support souple 3 de la matrice mais pas des transducteurs « haute fréquence » 4, obtenue par exemple au moyen d'une coque en résine,

- des moyens de connexion 5, par câble ou sans fil, au module 10 électronique de commande, ces moyens de connexion 5 pouvant être déconnectés et re-connectés du module 10 pour débrancher un premier type de sonde et re-brancher un deuxième type de sonde. Cela permet d'utiliser une sonde de « type 1 » ou « type2 », et cela permet en outre d'utiliser différentes tailles ou formes de sonde de « type 1 » ou différentes tailles ou formes de sonde de « type2 ».

Dans ce document, 1 MHz représente une fréquence d'1 méga Hertz, c'est-à-dire de $10^6$ Hertz.

Les moyens pour émettre des ondes ultrasonores sont de préférence en outre agencés pour émettre des ondes ultrasonores de fréquence inférieure à 4 MHz, de préférence entre 0,5MHz et 4MHz, la matrice 3, 4 ultrasonore souple comprenant en outre plusieurs transducteurs « basse fréquence » 4 agencés pour fonctionner en émission et en réception et agencés pour émettre des ondes ultrasonores de fréquence inférieure à 4 MHz, de préférence entre 0,5MHz et 4MHz (c'est-à-dire de préférence que chacun de ces transducteurs « basse fréquence » a une bande passante à -6dB Fsup-Finf, de sa réponse impulsionnelle, centrée sur une fréquence centrale (Finf+Fsup)/2 inférieure à 4 MHz, de préférence entre 0,5MHz et 4MHz), et montés sur le support souple 3 de sorte que chaque transducteur « basse fréquence » donné 4 soit agencé pour émettre une onde ultrasonore dans une direction 16 opposée et perpendiculaire à la surface du support souple 3 portant ce transducteur « basse fréquence » donné 4.

Par la suite de ce document, lorsque l'on parlera d'un ou des « transducteur(s) » (4), cela englobera par défaut le cas des transducteurs haute fréquence et des transducteurs basse fréquence.

On remarque que les transducteurs 4 « haute fréquence » et « basse fréquence » sont agencés pour fonctionner aussi en réception (pour recevoir leur écho pour la « haute fréquence » et recevoir par transmission l'onde émise par un autre transducteur situé sensiblement en face pour la « basse fréquence »). Les fonctions d'émission et de réception d'un transducteur 4 peuvent être scindées physiquement à deux endroits distincts, de sorte que pour les transducteurs à « basse fréquence » et/ou pour les transducteurs à « haute fréquence », la sonde selon l'invention peut comprendre une matrice d'émission entremêlée à une matrice de réception.

Par souple, on entend de préférence « qui se déforme sous son propre poids ». Par exemple, le support souple 3 seul (i.e. dénué de son armature 2) se déforme sous l'action de son propre poids, comme une feuille de papier : posé à plat sur une table, il prend une forme plate, mais soutenue par un de ses bords et éloigné au-dessus de cette table, il fléchit et se courbe sous l'action de son propre poids.

Par rigide, on entend de préférence « qui ne se déforme pas sous son propre poids ». Par exemple, l'armature rigide 2 seule ne se déforme pas sous l'action de son propre poids : posée sur une table, elle ne prend pas la forme plate de la table mais garde sa forme courbe ; soutenue par un de ses bords et éloignée au-dessus de cette table, elle garde toujours cette même forme courbe, sans déformation visible.

Toutefois, alors que les exemples ci-dessus concernent des formes développables par souci de simplification de l'explication, les formes souples et rigides concernées par l'invention peuvent être générées le cas échéant par des procédés 3D qui permettent la réalisation de formes anatomiques non développables, voire complètement gauches.

**La matrice ultrasonore souple 3 et 4**

Le support 3 est une plaque munie de deux faces 53, 54 opposées et parallèles. Sur une face 53 du support 3 sont montés les transducteurs 4, l'autre face 54 du support 3 est en contact avec l'armature 2.

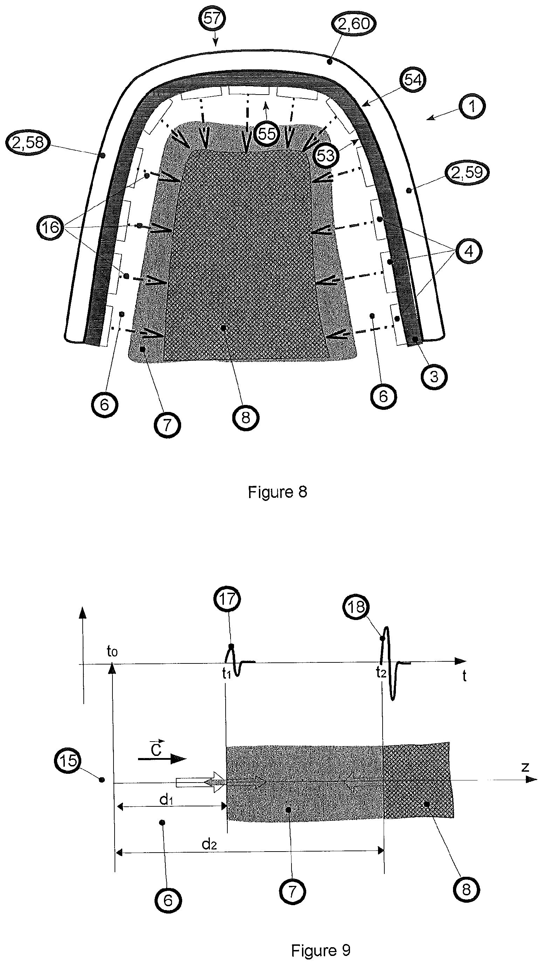

La multitude de petits transducteurs 4 sont fixés et répartis à la surface 53 du support 3 pour former une matrice souple d'éléments ultrasonores dont les axes 16 des faisceaux acoustiques générés par chaque élément sont perpendiculaires à la surface 53 du support (comme illustré sur la figure 8).

Le support 3 est situé sur la face arrière des transducteurs ultrasonores 4, l'émission ultrasonore se faisant sur la face avant des transducteurs qui est en contact avec le couplant acoustique 6.

Plusieurs configurations de matrice ultrasonore sont prévues.

Les transducteurs 4 sont de préférence des éléments électroacoustiques de type piézo-électrique ou autre.

Les transducteurs 4 sont de préférence disposés en une matrice de transducteurs élémentaires, linéaire à une dimension ou à deux dimensions formant une surface.

Le support souple 3 comprend de préférence un film de polymère (polyimide par exemple) ou en silicone ou un circuit imprimé flex agencé pour établir les connexions électriques.

La matrice ultrasonore 3, 4 peut par exemple être réalisée à partir d'une feuille de polymère ou copolymère piézo-électrique (PVDF, P(VDF-TrFE) ou autre) collée sur un circuit imprimé flexible (Flex) qui joue le rôle de support souple 3, en association avec le « backing » (milieu acoustique arrière) requis par le PVDF. Les éléments ultrasonores sont obtenus par sérigraphie de la métallisation sur la face émettrice du polymère piézo-électrique (face avant de la matrice) et par la sérigraphie du circuit imprimé.

La matrice ultrasonore peut aussi être réalisée en associant des matrices intégrées 1D ou 2D, cMUTs et/ou polymères ou copolymères piézo-électriques (PVDF...) et/ou composites et/ou céramiques piézo-électriques. Ces mini matrices sont issues des techniques du circuit intégré et peuvent comporter l'électronique de multiplexage et de pré-amplification. Elles sont assemblées sur un circuit imprimé souple (« Flex ») qui constitue le support formable 3 et qui assure les connexions électriques. Cette matrice peut aussi être réalisée en entremêlant des fibres optiques ou des guides optiques avec des petits transducteurs ultrasonores 4, de technologies précédemment citées, pour réaliser une matrice photo-acoustique. Les guides optiques émettent une impulsion optique dans la gencive parallèlement aux faisceaux acoustiques et les échos produits aux interfaces sont récupérées par les transducteurs ultrasonores 4 qui fonctionnent en récepteur, ceci dans le but de mieux discriminer le signal d'interface gencive/os.

Dans le cas où la sonde est utilisée pour faire des mesures en réflexion de l'épaisseur de la gencive afin de permettre la reconstruction du profil de l'os de la mâchoire, chaque élément génère une onde ultrasonore de fréquence comprise entre 10 et 25 MHz.

Dans un cas préférentiel, les transducteurs 4 peuvent comprendre des transducteurs 4 capables de fonctionner à des fréquences basses (0.5 à 4 MHz) et des transducteurs 4 capables de fonctionner à haute fréquence (10 à 25 MHz) qui sont :

- soit distincts, mais mélangés et répartis de manière identique sur la surface 53 du support 3,
- soit confondus, un même transducteur large bande pouvant aussi bien émettre des fréquences basses (0.5 à 4 MHz) et des hautes fréquences (10 à 25 MHz). Ceci est possible notamment avec la technologie des cMUTs (Dominique CERTON et al, PROCEDE ET DISPOSITIF DE GENERATION D'ULTRASONS METTANT EN OEUVRE DES CMUTS, ET PROCEDE ET SYSTEME D'IMAGERIE MEDICALE. FR2962926). Cette configuration permet de combiner des éléments basse fréquence pour réaliser des mesures en transmission et des éléments haute fréquence pour faire des mesures en réflexion de l'épaisseur de la gencive.

Ainsi, le dispositif 51 met de préférence en œuvre :

- d'une part, une technique spécifique d'imagerie par réflexion utilisant des ultrasons à haute fréquence (entre 10 et 25 MHz) pour déterminer, avec une résolution élevée, la position et la forme de l'os 8 situé sous la gencive 7, en effectuant des mesures de l'épaisseur de la gencive 7 en une multitude de points au moyen d'une matrice de transducteurs ultrasonores 4 positionnée autour de la zone concernée; on effectue ensuite une reconstruction en 3D de l'image à partir de ces mesures, éventuellement de la forme de la sonde 1, et éventuellement de l'image de l'anatomie externe de la cavité buccale. Ainsi la reconstruction 3D du profil de l'os 8 du maxillaire ou de la mandibule est obtenue à partir d'une mesure par réflexion de l'épaisseur de la gencive 7 avec des ultrasons de fréquence typiquement comprise entre 15 et 25 MHz. L'utilisation d'une telle gamme de haute fréquence et d'une matrice de transducteurs 4 pour multiplier les points de mesure permet d'améliorer considérablement la résolution de l'invention par rapport à l'état de l'art. La sonde 1 du dispositif 51 permet de faire des mesures en de nombreux points dont la position de chacun est connue précisément grâce à une calibration décrite ci-après.
- d'autre part, une technique utilisant des ultrasons de plus basse fréquence (entre 0.5 et 4 MHz) en transmission, pour définir la densité de l'os 8, très variable dans la bouche, ainsi que le positionnement des structures creuses incluses, comme les trajets nerveux ou les cavités sinusales. Une telle image obtenue en transmission avec des ultrasons de fréquences comprises entre 0.5 et 4MHz, permet de déterminer la position des structures sensibles incluses dans l'os 8, ainsi que les propriétés acoustiques (vitesse et atténuation) de l'os cortical et alvéolaire qui sont de bons indicateurs des propriétés mécaniques.

Chaque transducteur 4 a typiquement la forme d'une pastille (circulaire, carrée, rectangulaire, ...) ayant une aire d'approximativement 1mm$^2$ ou moins dans le cas d'une imagerie mode B

Pour la fabrication des transducteurs 4 sur support souple on peut se rapporter aux exemples suivants :

- « Design and Fabrication of a 40-MHz Annular Array Transducer », Jeffrey A. Ketterling et Al., IEE transactions on ultrasonics, ferroelectrics, and frequency control, vol. 52, N°4 April 2005,

- « Operational Vérification of a 40-MHz Annular Array Transducer », Jeffrey A. Ketterling et Al., IEE transactions on ultrasonics, ferroelectrics, and frequency control, vol. 53, N°3 March 2006,

- Thèse de Kuo-Ting Wu intitulée « development of Integrated and Flexible Ultrasonic Transducers for Aerospace Applications », McGill University, Montreal, Novembre 2010,
- « Flexible Transducer Arrays with through-wafer electrical interconnects based on trench refilling with PDMS », Xuefeng Zhuang, Der-Song Lin, Orner Oralklan, and butrus T. Khuri-Yakub, E. L. Ginzton Laboratory, Standford University, CA, USA, IEEE, 20th international conférences on MEMS, 2007, Hyogo Japan.
- « Flexible piezoelectric transducer for ultrasonic inspection of non-planar components », C. R. Bowen et al., Ultrasonics 48 (2008) p.367-375,
- « Simulation, Fabrication, and Characterization of a Novel Flexible, Conformai Ultrasound Transducer Array », Rahul S. Singh et al., 2007 IEEE Ultrasonics Symposium.

**L'armature rigide 2**

La matrice 3, 4 est rendue solidaire de l'armature rigide 2 qui est :

- soit initialement conformable puis susceptible d'être rigidifiée (résine silicone, lame formable, feuille de PVC thermo formable, etc.). Dans ce cas, les sondes sont configurables par le praticien en fonction de l'anatomie du patient. Dans le cas des sondes sur mesure, la matrice ultrasonore 3, 4 d'une sonde de « type 1 » doit pouvoir éventuellement être stérilisée et réutilisée. Dans ce cas, l'armature, adaptée à l'anatomie d'un patient donné, n'est pas réutilisable pour un autre patient.
- soit usinée directement dans sa forme définitive par un procédé de prototypage rapide (fraisage, imprimante 3D) dans un matériau rigide adapté, ou encore moulée sur un support anatomique (moulage de la mâchoire par exemple), dans une résine à polymérisation rapide ou équivalente, tous ces procédés étant connus de l'homme de l'art. Dans ce cas, les sondes sont réalisées en usine pour constituer une « panoplie » de sondes de tailles et de formes différentes. Les sondes de type 1 réalisées en usine sont en principe conçues pour être réutilisables et doivent donc pouvoir être stérilisées par un moyen approprié. Les sondes de type 2, qui sont positionnées à l'extérieur de la bouche, sont réutilisables.

Dans les deux cas, le principe de réalisation est le même, mais dans le cas d'une sonde configurable par le praticien, il faut prévoir une opération de calibration en cabinet, telle que décrite ci-après. Pour que la calibration soit figée, la matrice ultrasonore doit être préalablement rendue solidaire d'une armature rigide de même forme.

Un moyen de réalisation de l'armature 2 d'une sonde de type 1 consiste à couler une résine à polymérisation rapide à même le moulage en plâtre de la mâchoire ou en bouche, en intercalant la matrice souple ultrasonore 4,3 entre les deux.

D'autres moyens de réalisation font appel à un logiciel de dessin en 3D de cette armature 2 susceptible de piloter un dispositif de prototypage rapide.

La liaison ou fixation de la matrice ultrasonore 3, 4 (plus précisément du support souple 3) avec l'armature 2 est réalisée par clipsage ou au moyen d'un procédé mécanique, magnétique ou autre réversible. Ainsi, le support souple 3 est fixé (par exemple par clipsage ou vissage ou aimantation) à l'armature 2 de manière à être solidaire de l'armature 2 mais cette fixation est réversible (par exemple déclipsage ou dévissage ou éloignement des éléments aimantés) de manière à pouvoir être rendue amovible de l'armature rigide 2 pour permettre une interchangeabilité de l'armature rigide 2 parmi la « panoplie » de sondes de tailles et de formes différentes.

L'armature rigide 2 (et donc aussi le support souple 3 lorsqu'il est fixé à l'armature) a une forme courbe en « U » présentant un côté concave 55 entourant un axe central 56 et un côté convexe 57, la forme courbe étant délimitée par deux bords latéraux 58, 59 disposés face à face et reliés par un fond 60, le support souple 3 étant solidaire de l'armature rigide 2 du côté concave 55 de l'armature rigide 2.

Les extrémités des deux bords latéraux 58, 59 sont distants, du côté concave 55, d'une distance 61 comprise soit entre 8mm et 30mm pour une sonde de « type 1 » soit entre 4cm et 20cm pour une sonde de « type 2 ».

Chacun parmi les deux bords 58, 59 latéraux et le fond 60 de l'armature rigide 2 sont solidaires d'une partie de la matrice ultrasonore 3, partie sur laquelle sont montés des transducteurs 4.

**Le module électronique 10 (figure 7)**

Le module électronique 10 comporte un circuit d'émission 23 qui génère une impulsion électrique d'excitation. Cette impulsion est appliquée à un (ou plusieurs) élément(s) transducteur(s) 4 de la matrice 3, 4 via un système de sélection 24 (multiplexeur, démultiplexeur). Chaque transducteur 4 ainsi excité émet un faisceau ultrasonore haute fréquence (supérieure à 10MHz) ou basse fréquence (inférieure à 4MHz) selon l'impulsion électrique d'excitation et le type de transducteur. Tous les éléments transducteur(s) 4 peuvent ainsi être sélectionnés en émission individuellement ou en groupe, permettant de faire éventuellement une formation de faisceau d'ultrasons.

Certains des transducteurs 4 reçoivent ensuite en retour des ondes échos (fonctionnement en réception). Un ou plusieurs des signaux des échos 25, ainsi issus des transducteurs sélectionnés en réception 4, sont envoyés sur une chaine d'amplification 27 dont le gain est ajustable en fonction du temps (« Time Gain Control » ou TGC) via un circuit de sélection 26 (multiplexeur, démultiplexeur). Le signal en sortie de cette chaine d'amplification est numérisé par un numériseur 28 et envoyé au module de traitement des signaux 34, de type micro-ordinateur, via un circuit de liaison 29. Cette transmission série 11, 12 peut être de type USB ou Ethernet ou sans fil, WIFI par exemple.

Un circuit de contrôle 30 permet la sélection en émission 31 et en réception 32 des éléments transducteurs 4 de la sonde 1. Il assure le séquencement de l'acquisition 33 en relation avec l'opérateur via le circuit de liaison avec le module de traitement de signal 34 (type PC).

Il est parfaitement envisageable d'intégrer une partie, voir même l'intégralité de ce système électronique 10 dans la sonde 1, au plus près des éléments pour améliorer le rapport signal sur bruit (ou SNR ou « Signal to noise ratio ») et diminuer le câblage. Cette électronique permet d'assurer le balayage de l'ensemble des éléments transducteurs ultrasonores 4, afin de mesurer les dimensions des éléments biologiques (épaisseur de la gencive 7), de reconstruire une image ou analyser les signaux en vue de déterminer les propriétés acoustiques de l'os 8 avec les structures internes.

Il est à noter que le signal ultrasonore obtenu est complexe et constitué d'une multitude d'échos qui naissent dans le tissu gingivale. Un traitement du signal spécifique et plusieurs émissions ultrasonores successives (typiquement 10 à 5000, pour un moyennage satisfaisant) sont nécessaires pour améliorer le rapport signal sur bruit et déterminer précisément les positions des échos produits à l'entrée de la gencive 7 et sur l'os 8, et éliminer ainsi les artéfacts.

**Fonctionnement et utilisation de la sonde ultrasonore de « type 1 » (Figures 2, 3, 4, 5, 8, 9 et 10)**

Dans le cas de la sonde 1 de « type 1 », la matrice ultrasonore flexible 3, 4 est positionnée autour de la gencive 7 et du système de couplage acoustique 6.

La forme de la sonde 1 de « type 1 » est adaptée pour recouvrir la gencive 7 sur une zone édentée (comme illustré sur la figure 3 ou 4), ou les dents 36 et la gencive 7 avant extraction, ou sur une zone limitée à quelques dents (comme illustré sur la figure 5).

Pour la sonde de « type 1 », la matrice ultrasonore 3, 4 proprement dite est constituée d'un support flexible 3 :

- de forme rectangulaire lorsqu'il est mis à plat (comme illustré sur la figure 3), ou
- de forme rectangulaire avec deux découpes 62 symétriques lorsqu'il est mis à plat (comme illustré sur la figure 4) pour passer la crête de la gencive. Le fond 60 présente au moins une découpe 62 (deux sur la figure 4) qui rend sa largeur, mesurée selon une direction parallèle à l'axe central 56, inférieure à la largeur des bords latéraux 58, 59; ou
- de toute autre forme appropriée pour recouvrir une partie édentée ou non de la mâchoire.

Le principe de fonctionnement de la sonde 1 de « type 1 » en mode réflectif (émission de haute fréquence supérieure à 10MHz) est illustré sur les figures 8 et 9 et est le suivant (ce principe étant bien évidemment transposable à la sonde de « type 2 » lorsque celle-ci fonctionne en mode « haute fréquence ». Dans ce cas, l'image panoramique ainsi obtenue est limitée à la face antérieure de l'os et éventuellement des dents).

Les transducteurs « haute fréquence » 4 font chacun successivement une émission 15 d'une onde ultrasonore à un instant t0. Chaque onde ultrasonore émise par un transducteur « haute fréquence » 4 se propage dans une direction perpendiculaire 16 à la face 53, à la surface plane émettrice de ce transducteur « haute fréquence » et de préférence à la surface de la gencive 7. Le signal ultrasonore recueilli par le même transducteur « haute fréquence » 4 qui vient d'émettre est un signal de type mode A (pour « Amplitude Mode », ou « Mode Amplitude », mode de base en échographie, à distinguer d'un autre mode B classique d'échographie pour « Brightness mode » ou « mode brillance ») qui permet de déterminer la distance entre l'écho 17 produit à l'instant t1 à l'entrée de la gencive 7 et l'écho 18 produit à l'instant t2 par l'os 8, avec la relation suivante :

$$2(d2-d1)=C(t2-t1),$$

où C est la célérité des ultrasons (1500 à 1600 m.s$^{-1}$) et où d1 et d2 sont respectivement les distances entre le transducteur « haute fréquence » 4 venant d'émettre une onde ultrasonore et l'épithélium gingival 7, d'une part, et entre le transducteur « haute fréquence » 4 et l'interface gencive 7 / os 8, d'autre part. La différence, d2-d1, donne l'épaisseur de la gencive 7 sur l'os 8.

La gencive 7 étant relativement peu épaisse (généralement moins de 10 mm), on préfère utiliser des ultrasons de fréquence élevée, comprise entre 15 et 25 MHz, qui sont peu pénétrants et qui sont donc réfléchis en quasi-totalité par la surface de l'os cortical 8. L'avantage est d'obtenir des échos de courte durée, permettant de reconstruire des images 3D de résolution axiale élevée (meilleure que 100μm).

Le couplage acoustique entre la sonde 1 et la gencive 7 est assuré par une feuille souple de polymère hydrophile ou de gélatine 6 ou tout autre système permettant le transfert de l'énergie ultrasonore avec un minimum de pertes entre la face avant de la matrice souple 3 , 4 et la gencive 7. L'épaisseur, de l'ordre de quelques mm, compense les irrégularités de la gencive 7. Pour assurer un couplage acoustique optimal, le couplant doit être peu atténuant aux ultrasons et peut être enduit sur les deux faces de gel acoustique.

Pour permettre les mesures sur différentes morphologies de gencives 7, le dispositif 51 peut utiliser : soit une sonde 1 avec une armature formable par le praticien, soit un jeu de sondes 1 pré-calibrées qui possèdent des courbures et des largeurs préétablies. L'utilisation d'une sonde 1 à courbure formable par le praticien permet d'avoir un ajustement sur la gencive plus précis et surtout de limiter le nombre de sondes stockées pour répondre à la diversité des morphologies. Elle impose toutefois au praticien la réalisation d'une mesure supplémentaire sur une pièce ou forme de référence (ou « fantôme de calibration ») ou avec un scanner optique, par exemple laser, pour fournir au logiciel de reconstruction les paramètres de la géométrie de la sonde 1 ainsi que la position et l'axe d'émission de chaque transducteur (données de calibration) permettant d'établir la position des structures et de faire une reconstruction précise.

Lorsque cette sonde de « type 1 » comporte en outre des transducteurs basse fréquence générant des ondes capables de traverser l'os, elle peut fonctionner sur le principe d'un fonctionnement par transmission (et non par réflexion) comme expliqué sur la figure 10 et comme expliqué ci-après dans le cas d'une sonde de « type 2 ».

### Fonctionnement et utilisation de la sonde ultrasonore de « type 2 » (Figures 6 et 11)

Dans le cas de la sonde 1 de « type 2 », le principe est globalement le même mais la forme (toujours en « U ») de la sonde est légèrement différente notamment au niveau de ses dimensions qui sont plus grandes car le champ de mesure est plus important.

La sonde de « type 2 » est destinée à pratiquer une tomographie globale de la mâchoire par transmission (comme illustré sur les figures 6 et 11), ce qui nécessite des ultrasons de basse fréquence dont la fréquence est comprise entre 0,5 et 4 MHz.

Elle est positionnée à l'extérieur de la cavité buccale (joues, menton), et possède une forme en U qui englobe la totalité de la mandibule ou du maxillaire (comme illustré sur la figure 6). Elle nécessite l'application sur la peau du visage d'un gel de couplage acoustique 6, et la mise en bouche d'un dispositif de couplage acoustique 6 qui peut être du gel acoustique et/ou de la gélatine voire même de l'eau.

Cette sonde 1 est réalisée suivant le même principe que celle de « type 1 », mais sa surface est plus grande et sa résolution est inférieure en raison d'une fréquence ultrasonore plus basse, par exemple de 0,5 à 4 MHz, des distances plus grande avec l'os, et d'une utilisation en mode tomographique par transmission.

Elle permet aussi une description panoramique de la forme de l'os 8 en mode réflectif haute fréquence, par exemple de 10 à 15MHz, sur le principe décrit précédemment dans le cas de la sonde de « type 1 ».

Comme pour la sonde de « type 1 », sa forme peut être fixe et configurée en usine, ou adaptable par le dentiste à l'anatomie du patient. Les considérations concernant la réalisation pratique de la sonde de « type 1 » s'appliquent donc aussi à la sonde de « type 2 ».

Du fait de son positionnement extérieur à la cavité buccale (sur la partie inférieure 13 ou supérieure 14 de l'extérieur de la bouche), la sonde de « type 2 » est plus accessible, et sa capacité à couvrir la totalité de l'arcade maxillaire ou mandibulaire la rend complémentaire de la sonde de « type 1 ».

### Utilisations combinées des deux types de sonde

On aura donc tendance à utiliser les deux types de sondes à l'intérieur et à l'extérieur d'une même bouche selon le procédé suivant :

- une sonde de « type 1 » est montée à l'intérieur de la bouche en recouvrant une gencive 7 sur une zone édentée à l'intérieur de cette bouche ou recouvrant une ou plusieurs dents 36 et leur gencive 7 à l'intérieur de cette bouche; des transducteurs 4 de cette sonde de « type 1 » émettent des ondes ultrasonores de fréquence haute (d'au moins 10 MHz, de préférence d'au moins 15 MHz, de préférence comprise entre 15MHz et 25 Mhz) ou basse (inférieure à 4 Mhz, de préférence comprise entre 0.5MHz et 4MHz), de préférence de fréquence haute ;
- une sonde de « type 2 » est placée à l'extérieur de cette même bouche en entourant tout ou partie d'un arc mandibulaire ou maxillaire de cette bouche, ou en même temps des deux parties d'une mâchoire située à l'intérieur de cette bouche ; des transducteurs 4 de cette sonde de « type 2 » émettent des ondes ultrasonores de fréquence haute (d'au moins 10 MHz, de préférence comprise entre 10MHz et 15 Mhz) ou basse (inférieure à 4 Mhz, de préférence comprise entre 0.5MHz et 4MHz), de préférence de fréquence basse ;

l'utilisation de deux sondes de « type 1 » et « type 2 » étant possible grâces à leurs moyens de connexion 5 qui sont déconnectables et reconnectables aux moyens de commande 10, ce qui permet de switcher entre les deux types de sonde.

Cela permet d'obtenir, avec les fréquences basses, une tomographie en 3D de l'os cortical et alvéolaire de tout ou partie de la mâchoire, avec leurs structures internes, et de définir leur densité moyenne et/ou ponctuelle en tout point de la zone.

Les ondes ultrasonores de hautes fréquences permettent d'obtenir, en mode réflectif, une mesure précise du profil de l'os cortical sur sa face antérieure.

**Le traitement des signaux par le module 34**

Les signaux en provenance des transducteurs et acquis par la module 10 d'acquisition de données sont traités au sein du module 34 de traitement de données, typiquement par un logiciel mettant en œuvre un procédé de traitement de ces signaux.

Pour un fonctionnement en réflexion en haute fréquence, par exemple pour la sonde de « type 1 » de la figure 8, le logiciel effectue une reconstruction du profil de l'os 8 situé sous la gencive 7, à partir des signaux transmis par le module électronique 10, des données de calibration de la sonde, et, éventuellement, des données 3D définissant la forme de la gencive 7. Les signaux transmis par le module électronique 10 consistent alors en des mesures, en de nombreux points, de la distance entre la sonde 4 et l'os cortical 8, qui permettent de déterminer l'épaisseur de la gencive 7.

Lorsque la sonde 1 (de « type 1 » ou « type 2 ») travaille en basse fréquence (comme illustré sur la figure 10 pour le cas d'une sonde de « type 1 » ou comme illustré sur la figure 11 pour le cas d'une sonde de « type 2 »), le logiciel est capable de traiter les signaux transmis à travers l'os 8 de la mâchoire pour en extraire une représentation paramétrique de sa structure interne, par une technique de tomodensitométrie ultrasonore (« A prototype of 500 kHz ultrasonic matricial device : beam scanner » Marielle Defontaine et al., 1999 IEEE Ultrasonics Symposium ; « Expérimental évaluation of bone quality measuring speed of sound in cadaver mandibles », Oyad Al Haffar et al., Oral Surgery, Oral Medicine, Oral Pathology, Oral Radiology, and Endodontology, vol.102, n°6, december 2006 ; «In vivo ultrasound assessment of skeletal status : principles and techniques », P. laugier, Journées Os-Ultrasons, Compiègne 24-25 janvier 2002 ; « The measurements of ultrasound parameters on calcaneus by two-sided interrogation techniques », Pei-Jarn Chen et al., Measurement Science and technology, vol. 16, 2005).

Pour une sonde de « type 2 », le logiciel traite principalement des signaux issus de la transmission basse fréquence des ondes ultrasonores à travers la mâchoire et la bouche, afin de réaliser une reconstruction de type tomographie ou scanner. Il traite aussi les signaux réflectifs de haute fréquence émis par la sonde, afin de réaliser un profil 3D des os de l'arc maxillaire ou mandibulaire.

Le logiciel est composé de plusieurs programmes dont les fonctionnalités complémentaires sont utiles à un chirurgien dentiste, par exemple dans le cadre de la pose d'implants dentaires.

La principale fonctionnalité est de traiter les données fournies par le système électronique 10 afin de réaliser des images 3D des structures osseuses de la mâchoire, et d'en extraire des informations sur leur densité.

Elle est assurée par un programme de détection des échos produits :

- en haute fréquence, aux interfaces de la gencive 7 et de l'os 8, afin de déterminer le profil de celui-ci (signaux en mode A localisés dans l'espace) ;
- en basse fréquence, par les faisceaux ultrasonores traversant l'os 8 afin de permettre la reconstruction en 3D des structures internes, de mesurer les paramètres acoustiques de vitesse (célérité) et d'atténuation pour déterminer des paramètres de densité osseuse.

Les données brutes étant généralement très bruitées, un programme dit « de segmentation » est nécessaire pour fournir les informations pertinentes pour un praticien.

La mise en forme des signaux provenant du système électronique 10 associe à chacun d'eux une géométrie 3D (point source et direction), issue de la procédure de calibration. Ainsi, les données initiales (point d'émission d'ultrasons, direction d'émission et distance d'écho) permettent de déterminer un nuage de point correspondant à l'objet imagé par les ultrasons. Le logiciel, par extraction des paramètres dimensionnels de la sonde 1, permet de générer une image 3D. Le logiciel tient compte des vitesses des ultrasons en fonction des milieux traversés.

Ce type de reconstruction 3D est connu (thèse intitulée « A novel Imaging System for Automatic Real-Time 3D patient-Specific Knee Model Reconstruction Using Ultrasound RF Data »).

Le bruit des images échographiques étant générateur de multiples contours parasites, l'extraction des surfaces 3D à partir de ces signaux peut nécessiter d'introduire de la « connaissance a priori » dans le logiciel. Ces méthodes sont connues de l'homme de l'art. Leur application nécessite un traitement lié à la connaissance des artefacts dus aux échos de répétition dans des structures stratifiées, ou l'utilisation de modèles avec combinaisons multiples gérant les particularités de la zone concernée à partir d'un modèle osseux déformable mais unique.

Ces images 3D sont destinées à être utilisées dans des cabinets dentaires, en remplacement des images de scanners RX, notamment lors de la pose d'implants assistée par informatique.

Le dispositif 51 peut être :

- employé seul dans un mode « ultrasons seuls »,
- ou associé, dans une configuration multimodale, à d'autres techniques complémentaires d'imagerie classiques, peu ou pas ionisantes.

Dans le mode « ultrasons seuls », le niveau d'irradiation des patients est ramené à zéro.

Dans la configuration « multimodale », des images issues selon l'invention sont combinées informatiquement avec des images d'au moins une autre technique complémentaire pour donner naissance à une image 3D composite de la gencive 7, des dents 36 et de l'os 8, cortical et alvéolaire, du maxillaire ou de la mandibule. La contribution de ces techniques complémentaires est adaptée suivant les cas. Ainsi, cette au moins une autre technique complémentaire peut par exemple comprendre:

- un scanner optique de la cavité buccale, qui fournit une prise d'empreinte numérique 3D avec une précision pouvant atteindre $20\mu$m, permet d'établir des références anatomiques utilisées pour obtenir une définition spatiale précise de l'image ultrasonore ;
- des images rétro-alvéolaires et panoramiques RX, numérisées, sont fusionnées avec les images ultrasonores pour préciser la localisation des structures incluses sensibles, comme le nerf dentaire ou le plancher du sinus, ainsi que la densité de l'os.

L'intégration des données ultrasonores dans un ensemble multimodal mettant en oeuvre d'autres techniques d'imagerie (radios rétro alvéolaires et panoramiques, numérisation des surfaces externes,...), est prise en compte. Cela implique notamment que les formats des images issues du dispositif ultrasonore soient compatibles avec ceux des images fournies par les autres techniques, notamment les images au format DICOM, couramment utilisé en imagerie dentaire, et aux autres formats susceptibles d'être rencontrés (STL,...).

Dans le cas d'une utilisation multimodale, faisant appel à d'autres techniques d'imagerie, le logiciel effectue la fusion entre l'image ultrasonore 3D et une image issue d'une ou de plusieurs autres techniques évoquées précédemment. Ces opérations de mixage d'images sont bien connues de l'homme de l'art.

Un programme permet d'évaluer la densité osseuse relative (unités Hounsfield) moyenne ou ponctuelle dans les différentes zones concernées. Dans le cadre du protocole multimodal, les données sur la qualité de l'os fournies par d'autres sources sont fusionnées avec celles du dispositif ultrasonore.

Le logiciel comporte par ailleurs un programme de dessin en 3D de l'armature de la matrice acoustique. Ce dessin est, soit réalisé à partir du programme de calibration, soit issu directement de l'image de la partie concernée fournie par une caméra optique 3D. Il représente typiquement une armature 2 qui est réalisée à l'aide d'une imprimante 3D, ou d'une machine de fraisage numérique, l'une et l'autre étant pilotées par le logiciel de dessin de la sonde. L'usage de ces sondes n'étant pas chirurgical, elles peuvent être réalisées dans des résines non biocompatibles, telles que l'ABS ordinaire.

**Application à la planification de la pose d'implants**

Le dispositif fournit les images 3D et, plus généralement, les informations nécessaires à un dentiste utilisant un logiciel de planification d'implants dentaires pour préparer sa chirurgie dans la région étudiée. Le logiciel du dispositif d'imagerie 51 doit donc pouvoir s'interfacer avec un logiciel de planification existant sur le marché, en fournissant des images aux formats utilisés en imagerie médicale.

Comme évoqué précédemment, la sonde ultrasonore de « type 1 » conformée sur mesure au cabinet dentaire présente de grandes similitudes de forme avec le guide chirurgical lui-même. De ce fait, le dessin en 3D d'un tel guide peut être réalisé au moyen d'un autre programme de dessin 3D, associant le dessin de l'armature de la sonde, aux données fournies par un logiciel de planification implantologique, position et axes des cylindres de guidage des forets notamment.

La fabrication du guide, habituellement confiée à une usine spécialisée ou à un laboratoire de prothèses, peut alors être effectuée au cabinet, suivant le même procédé que l'armature de la sonde, mais en utilisant une résine bio compatible. L'ensemble du procédé améliore considérablement les coûts et les délais de la pose d'implants assistée par informatique.

Le dispositif d'imagerie 3D objet de ce brevet peut également être associé au pilotage de systèmes de guidage virtuels dits « de navigation ».

En pratique, on peut imaginer plusieurs types d'appareils commercialisables. En voici deux exemples :

- Pour les centres de radiologie, hospitaliers et privés, et les gros cabinets dentaires, un appareil autonome, similaire à celui proposé par Atys médical en dermatologie, éventuellement portable, comme l'Orcheolite de Sonoscanner. Toutes les fonctionnalités décrites dans le brevet sont proposées par cet appareil.
- Pour le cabinet dentaire moyen, un matériel simplifié au maximum, pouvant se réduire à une série de sondes de « type 1 », de tailles et de formes identifiées, ou à une sonde conformable pouvant être calibrée sur un modèle 3D de la mâchoire du patient ; ces sondes sont reliées au port USB d'un PC de bureau équipé d'une carte spécifique de traitement des images, la sortie vidéo se faisant sur l'écran du PC, et les copies papier sur l'imprimante associée au PC.

**Calibration de la courbure et de la forme de la sonde 1**

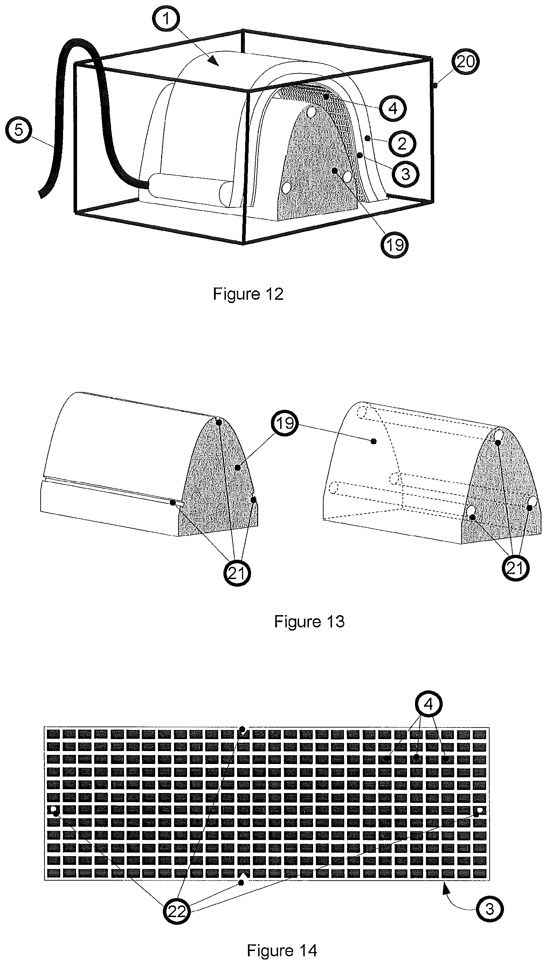

Pour permettre une reconstruction précise des structures, le logiciel doit connaître les données de calibration, c'est-à-dire pour chaque transducteur 4 composant la matrice ultrasonore 3, 4 la position spatiale des faisceaux acoustiques (point source et direction). Cette information est obtenue par une mesure sur un fantôme de calibration 19 (c'est-à-dire un gabarit illustré sur la figure 12, typiquement plongé dans une cuve 20 remplie d'un liquide de couplage) ou en utilisant un scanner optique, laser ou autre, ou une caméra 3D intraorale. Pour cela, on fait fonctionner des transducteurs 4 de la sonde 1 en émission et en réception au sein d'un dispositif selon l'invention alors que la matrice ultrasonore souple 3,4 entoure le gabarit 19 plongé dans un liquide de couplage.

Lorsque la courbure de l'armature 2 et donc du support 3 est prédéfinie en usine, les paramètres ou données de calibration sont automatiquement fournis au logiciel (mémoire incluse dans la sonde 1 ou dans un fichier paramètres).

Lorsque la courbure de l'armature 2 et donc du support 3 est réalisée par le praticien sur la mâchoire du patient, ou sur un moulage en plâtre de celle-ci, la calibration (figure 12) est réalisée au cabinet, par exemple sur un fantôme de calibration 19 (typiquement en matière plastique) situé dans une petite cuve 20 contenant un liquide de couplage acoustique (typiquement de l'eau, comprenant éventuellement en outre un antiseptique et/ou un antifongique). Le fantôme de calibration 19 a une forme parfaitement connue et possède des repères acoustiques 21 tel (s) que creux, trou(s), bosse(s), rainure(s), et/ou inclusion(s) (rainure sur la surface extérieure du gabarit 19 dans l'exemple à gauche de la figure 13, ou trous (cylindriques) à l'intérieur du gabarit dans l'exemple à droite de la figure 13) qui permettent d'identifier la position des transducteurs 4 en regard avec ces repères. Cette information est nécessaire pour connaitre la position dans l'espace de chaque transducteur 4 de la matrice souple 3. Dans le cas d'une mesure de la courbure par une autre méthode, caméra optique ou autre, la matrice souple 3, 4 possède plusieurs repères 22 adaptés (figure 14) à la méthode de mesure (trou, encoche, sérigraphie ou autre) permettant de déterminer précisément la position de chaque transducteur 4 dans l'espace.

Un programme de calibration est alors intégré au logiciel du dispositif.

En résumé, le dispositif 51 est un :

1) dispositif ultrasonore de résolution suffisante pour pouvoir être installé et exploité de manière intéressante par un praticien dans un cabinet dentaire ;
2) un dispositif pouvant être utilisé en tant qu'appareil d'imagerie 3D autonome, ou pouvant être associé à d'autres systèmes d'imagerie peu ionisants, présents au cabinet (radiographie panoramique ou rétroalvéolaire numérisée, scanner optique), ou autre, pour fournir au dentiste les images lui permettant de planifier sa chirurgie ;
3) un dispositif non irradiant,
4) un dispositif d'un prix inférieur à ceux des appareils d'imagerie 3D du marché, y compris des CBCTs.

Bien sûr, l'invention n'est pas limitée aux exemples qui viennent d'être décrits et de nombreux aménagements peuvent être apportés à ces exemples sans sortir du cadre de l'invention.

Par exemple, la sonde 1 peut intégrer tout ou partie de l'électronique 10 de contrôle, d'excitation et de pré-amplification de la matrice ultrasonore souple, et/ou tout ou partie de la numérisation des données analogiques et leur transmission, et ou tout ou partie de l'électronique 34.

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Sonde ultrasonore (1) d'imagerie dentaire, comprenant:

   des moyens pour émettre des ondes ultrasonores, agencés pour émettre des ondes ultrasonores de fréquence d'au moins 10 MHz, ces moyens d'émission d'ondes ultrasonores comprenant une matrice ultrasonore souple (3, 4) comprenant:

   > un support souple (3); et
   > plusieurs transducteurs (4) agencés pour fonctionner en émission et en réception dans un mode de réflexion, agencés pour émettre des ondes ultrasonores de fréquence d'au moins 10 MHz, et montés sur le support souple et disposés sous forme de matrice;
   > une armature rigide (2) solidaire et en contact avec la matrice ultrasonore souple (3, 4), de sorte que chaque transducteur (4) donné soit agencé pour émettre une onde ultrasonore dans une direction opposée à l'armature rigide(2).

2. Sonde ultrasonore selon la revendication 1, dans laquelle le support souple (3) est monté de manière amovible dans l'armature rigide (2) pour une interchangeabilité de l'armature rigide (2).

3. Sonde ultrasonore selon l'une quelconque des revendications précédentes, dans laquelle l'armature rigide (2) a une forme courbe présentant un côté concave (55) entourant un axe central (56) et un côté convexe (57), la forme courbe étant définie par deux bord latéraux (58; 59) disposés face à face et reliés par un fond (60), le support souple (3) étant solidaire de l'armature rigide (2) du côté concave (55) de l'armature rigide.

4. Sonde ultrasonore selon la revendication précédente, **caractérisée en ce que** les deux bords latéraux (58,59) sont distants, du côté concave (55), d'une distance (61) comprise soit entre 8 mm et 30 mm soit entre 4cm et 20 cm.

5. Sonde ultrasonore selon la revendication 3 ou 4, **caractérisée en ce que** le fond présente au moins une découpe (62) qui rend sa largeur, mesurée selon une direction parallèle à l'axe central (56), inférieure à la largeur des bords latéraux (58, 59).

**6.** Sonde ultrasonore selon l'une quelconque des revendications précédentes, comprenant en outre plusieurs guides optiques montés sur le support souple (3) et destinés à émettre une impulsion optique dans la gencive parallèlement aux faisceaux acoustiques générés par les transducteurs (4), les guides optiques étant entrelacés avec les transducteurs (4) pour réaliser une matrice photo-acoustique.

**7.** Sonde ultrasonore selon l'une quelconque des revendications précédentes, **caractérisée en ce que** les transducteurs (4) sont des éléments électroacoustiques ou opto-acoustiques disposés en une matrice de transducteurs élémentaires, linéaire à une dimension ou à deux dimensions formant une surface, ou à trois dimensions formant une surface ou un volume.

**8.** Sonde ultrasonore selon l'une quelconque des revendications précédentes, dans laquelle la matrice souple de transducteurs (3, 4) comprend en outre une pluralité de repères (22) détectables pour déterminer une position 3D de chaque transducteur (4) sur le support souple (3).

**9.** Sonde ultrasonore selon la revendication 8, dans laquelle la pluralité de repères détectables comprend des trous, des encoches ou de la sérigraphie.

**10.** Dispositif (51) d'imagerie dentaire, comprenant :

- une sonde (1) selon l'une quelconque des revendications précédentes,
- des moyens (10) de commande de la sonde, et
- des moyens (34) de traitement de signaux provenant de la sonde (1), dans laquelle les moyens (10) de commande de la sonde sont agencés pour opérer dans un mode de réflexion utilisant les plusieurs transducteurs (4) pour émettre des ondes ultrasonores de fréquence d'au moins 10 MHz, pour générer une image 3D des os de la mâchoire.

**11.** Dispositif d'imagerie dentaire selon la revendication 10, dans laquelle les moyens (10) de commande de la sonde sont agencés pour opérer en outre dans un mode transmissif utilisant les transducteurs (4) pour émettre des ondes ultrasonores de fréquence inférieure à 4 MHz, pour générer une reconstruction 3D de structures internes d'une ou plusieurs dents.

**12.** Procédé d'utilisation d'un dispositif selon la revendication 10 ou 11, dans laquelle une sonde (1) selon l'une quelconque des revendications 1 à 9 est montée à l'intérieur d'une bouche en recouvrant une gencive (7) sur une zone édentée ou recouvrant une ou plusieurs dents et leur gencive (7), ou placée à l'extérieur d'une bouche en entourant tout ou partie d'un arc mandibulaire ou maxillaire, ou deux parties d'une mâchoire en même temps, le procédé comprenant l'étape suivante:
émettre des ondes ultrasonores de fréquence d'au moins 10 MHz dans un mode de réflexion pour générer une image 3D des os de la mâchoire.

**Patentansprüche**

**1.** Eine Ultraschallsonde (1) zur dentalen Bildgebung, die Folgendes aufweist:
Mittel zum Emittieren von Ultraschallwellen, die konfiguriert sind zum Emittieren von Ultraschallwellen mit einer Frequenz von wenigstens 10 MHz, wobei die Mittel zum Übertragen der Ultraschallwellen eine flexible Ultraschallmatrix (3, 4) besitzen, die Folgendes aufweist:

einen flexiblen Träger (3);
eine Vielzahl von Wandlern (4), die konfiguriert sind zum Betrieb für Emission und Rezeption von Ultraschallwellen in einem Reflexionsmodus, zum Emittieren von Ultraschallwellen mit einer Frequenz von wenigstens 10 MHz, und die an dem flexiblen Träger (3) angebracht und zum Bilden der Matrix angeordnet sind;
einen starren Halter (2), der verbunden und in Kontakt mit der flexiblen Ultraschallmatrix (3, 4) ist, so dass jeder Wandler (4) angeordnet ist zum Übertragen einer Ultraschallwelle in einer Richtung weg von dem starren Halter (2).

**2.** Ultraschallsonde nach Anspruch 1, wobei der flexible Träger (3) in einer lösbaren Art und Weise an dem starren Halter (2) zum Austausch des starren Halters angebracht ist.

**3.** Ultraschallsonde nach einem der vorhergehenden Ansprüche, wobei der starre Halter (2) eine gebogene Form besitzt mit einer konkaven Seite (55), die eine Mittelachse (56) umgibt, und einer konvexen Seite (57), wobei die gebogene Form durch zwei Seitenteile (58, 59) definiert ist, die zueinander weisend angeordnet sind und durch einen Boden (60) verbunden sind, und wobei der flexible Träger (3) mit dem starren Halter (2) an der konkaven Seite (55) des starren Halters verbunden ist.

**4.** Ultraschallsonde nach einem der vorhergehenden Ansprüche, **dadurch gekennzeichnet, dass** die zwei Seitenteile (58, 59) von der konkaven Seite (55) mit einem Abstand (61) von entweder 8 mm bis 30 mm oder 4 cm bis 20 cm beabstandet sind.

**5.** Ultraschallsonde nach Anspruch 3 oder 4, **dadurch gekennzeichnet, dass** der Boden wenigstens einen Ausschnitt (62) besitzt, so dass dessen Breite gemessen in einer Richtung parallel zu der Mittelachse (56) geringer als die Breite der Seitenteile (58, 59) ist.

6. Ultraschallsonde nach einem der vorhergehenden Ansprüche, die ferner eine Vielzahl von optischen Leitern aufweist, die an dem flexiblen Träger (3) angebracht sind und konfiguriert sind zum Emittieren eines optischen Impulses an das Zahnfleisch, und zwar parallel zu dem akustischen Strahl, der von den Wandlern (4) erzeugt wird, wobei die optischen Leitern mit den Wandlern (4) verschachtelt angeordnet sind, um eine fotoakustische Matrix zu bilden.

7. Ultraschallsonde nach einem der vorhergehenden Ansprüche, **dadurch gekennzeichnet, dass** die Wandler (4) elektroakustische Elemente oder optoakustische Elemente sind, die in der Matrix der elementaren Wandler angeordnet sind, und zwar entweder linear eindimensional oder zweidimensional eine Oberfläche bildend oder dreidimensional eine Oberfläche oder ein Volumen bildend.

8. Ultraschallsonde nach einem der vorhergehenden Ansprüche, wobei die flexible Wandlermatrix (3, 4) ferner eine Vielzahl von detektierbaren Markierungen (22) zum Bestimmen einer 3D-Position jedes Wandlers (4) an dem flexiblen Träger aufweist.

9. Ultraschallsonde nach Anspruch 9, wobei die detektierbaren Markierungen Stifte, Löcher, Einschnitte oder Siebdruck aufweisen.

10. Dentale Abbildungsvorrichtung (51), die Folgendes aufweist:

eine Sonde (1) nach einem der vorhergehenden Ansprüche,
Steuermittel (10) zum Steuern der Sonde, und Mittel (34) zum Verarbeiten der Signale, die von der Sonde bereitgestellt werden,
wobei die Steuermittel (10) zum Steuern der Sonde konfiguriert sind zum Betrieb in einem ersten Reflexionsmodus unter Verwendung der Wandler (4) zum Emittieren von Ultraschallwellen mit einer Frequenz von wenigstens 10 MHz zum Erzeugen eines 3D-Bildes des Kieferknochens.

11. Dentale Abbildungsvorrichtung nach Anspruch 10, wobei die Steuermittel (10) zum Steuern der Sonde ferner konfiguriert sind zum Betrieb in einem Transmissionsmodus unter Verwendung der Wandler (4) zum Emittieren von Ultraschallwellen mit einer Frequenz unter 4 MHz zum Erzeugen einer 3D-Rekonstruktion der internen Strukturen eines Zahnes oder mehrerer Zähne.

12. Verfahren zum Verwenden einer Vorrichtung nach Anspruch 10 oder 11, wobei eine Sonde (1) nach einem der Ansprüche 1 bis 9 innerhalb eines Mundes derart angeordnet wird, das sie das Zahnfleisch (7) in einem zahnlosen Bereich abdeckt, oder einen Zahn oder mehr Zähne und das Zahnfleisch (7) abdeckt, oder wobei die Sonde außerhalb des Mundes derart angeordnet wird, um das Ganze oder Teile des oberen Dentalbogens oder des unteren Dentalbogens oder beide Bereiche des oberen und unteren Dentalbogens zur gleichen Zeit zu umschließen, wobei das Verfahren die folgenden Schritte aufweist: Emittieren von Ultraschallwellen mit einer Frequenz von wenigstens 10 MHz in einem Reflexionsmodus zum Erzeugen eines 3D-Bildes des Kieferknochens.

**Claims**

1. An ultrasonic probe (1) for dental imagery, comprising:
means for emitting ultrasonic waves, configured to emit ultrasonic waves at a frequency of at least 10 MHz, wherein the means for transmitting ultrasonic waves has a flexible ultrasonic matrix (3, 4) comprising:

a flexible support (3);
a plurality of transducers (4) configured to operate for emission and reception in a reflection mode, emit ultrasonic waves at a frequency of at least 10 MHz, and mounted to the flexible support (3) and arranged to from the matrix;
a rigid holder (2) conjoined and in contact with the flexible ultrasonic matrix (3, 4), so that each transducer (4) is arranged to transmit an ultrasonic wave in a direction away from the rigid holder (2).

2. The ultrasonic probe of claim 1, wherein the flexible support (3) is mounted in a detachable manner to the rigid holder (2) for replacement of the rigid holder.

3. The ultrasonic probe of any one of the preceding claims, wherein the rigid holder (2) has a curved form having a concave side (55) surrounding a central axis (56) and a convex side (57), the curved form being defined by two side portions (58, 59) arranged face to face and connected by a bottom (60), the flexible support (3) being conjoined with the rigid holder (2) on the concave side (55) of the rigid holder.

4. The ultrasonic probe according to any one of preceding claims, **characterized in that** the two side portions (58, 59) are separated from the concave side (55), at a distance (61) of either between 8mm and 30mm or between 4cm and 20cm.

5. The ultrasonic probe according to claim 3 or 4, **char-**

**acterized in that** the bottom has at least one cutout (62) such that its width, measured in a direction parallel to the central axis (56), is less than the width of the side portions (58, 59).

6. The ultrasonic probe according to any of the preceding claims, further comprising a plurality of optical guides mounted to the flexible support (3) and configured to emit an optical impulse to the gum in parallel to the acoustic beam generated by the transducers (4), wherein the optical guides are interlaced with the transducers (4) to form a photo-acoustic matrix

7. The ultrasonic probe according to any of the preceding claims, **characterized in that** the transducers (4) are electro-acoustic elements or opto-acoustic elements arranged in matrix of elementary transducers, either linearly in one-dimension or two-dimensions, forming a surface or in three dimensions forming a surface or volume.

8. The ultrasonic probe according to any of the preceding claims, wherein the flexible transducer matrix (3, 4) further comprises a plurality of detectable markings (22) for determining a 3D position of each transducer (4) on the flexible support.

9. The ultrasonic probe according to claim 8, wherein the detectable markings comprises pins, holes, notches, or serigraphy

10. Dental imaging device (51), comprising:

   a probe (1) according to any one of the preceding claims,
   control means (10) for controlling the probe, and means (34) for processing the signals provided by the probe (1),
   wherein the control means (10) for controlling the probe is configured to operate in a reflection mode, using the plurality of transducers (4) for emitting the ultrasonic waves having the frequency of at least 10 MHz for generating a 3D image of the jawbone.

11. Dental imaging device according to claim 10, wherein the control means (10) for controlling the probe is further configured to operate in a transmission mode, using the transducers (4) for emitting ultrasonic waves having a frequency below 4 MHz for generating a 3D reconstruction of the internal structures of one tooth or more teeth.

12. A method for using a device according to Claim 10 or 11, wherein a probe (1) according to any one of claims 1 to 9 is mounted inside a mouth covering the gum (7) at a toothless area or covering one or more teeth and the gum (7) or wherein the probe is placed outside the mouth to surround the whole or part of an upper dental arch or a lower dental arch, or both regions of the upper and lower dental arch at the same time, wherein the method comprises the following steps:
emitting ultrasonic waves having a frequency of at least 10 MHz in a reflection mode for generating a 3D image of the jawbone.

Figure 1

Figure 2

Figure 3

Figure 4

Figure 5

Figure 6

Figure 7

Figure 8

Figure 9

Figure 10

Figure 11

Figure 12

Figure 13

Figure 14

## RÉFÉRENCES CITÉES DANS LA DESCRIPTION

**Documents brevets cités dans la description**

- US 7285093 B2
- US 20120244489 A1
- FR 2962926

**Littérature non-brevet citée dans la description**

- Ultrasonic détermination of thickness of masticatory mucosa. *Oral Surgery Oral Medicine Oral Pathology,* Août 1999, vol. 88 (2
- **JEFFREY A. KETTERLING et al.** Design and Fabrication of a 40-MHz Annular Array Transducer. *IEE transactions on ultrasonics, ferroelectrics, and frequency control,* Avril 2005, vol. 52 (4
- **JEFFREY A. KETTERLING et al.** Operational Vérification of a 40-MHz Annular Array Transducer. *IEE transactions on ultrasonics, ferroelectrics, and frequency control,* Mars 2006, vol. 53 (3
- **KUO-TING WU.** development of Integrated and Flexible Ultrasonic Transducers for Aerospace Applications. McGill University, Novembre 2010
- Flexible Transducer Arrays with through-wafer electrical interconnects based on trench refilling with PDMS. **XUEFENG ZHUANG ; DER-SONG LIN ; ORNER ORALKLAN ; BUTRUS T. KHURI-YAKUB.** IEEE, 20th international conférences on MEMS. Standford University, 2007
- **C. R. BOWEN et al.** Flexible piezoelectric transducer for ultrasonic inspection of non-planar components. *Ultrasonics,* 2008, vol. 48, 367-375
- **RAHUL S. SINGH et al.** Simulation, Fabrication, and Characterization of a Novel Flexible, Conformai Ultrasound Transducer Array. *2007 IEEE Ultrasonics Symposium*
- **MARIELLE DEFONTAINE et al.** A prototype of 500 kHz ultrasonic matricial device : beam scanner. *1999 IEEE Ultrasonics Symposium*
- **OYAD AL HAFFAR et al.** Expérimental évaluation of bone quality measuring speed of sound in cadaver mandibles. *Oral Surgery, Oral Medicine, Oral Pathology, Oral Radiology, and Endodontology,* Décembre 2006, vol. 102 (6
- **P. LAUGIER.** In vivo ultrasound assessment of skeletal status : principles and techniques. *Journées Os-Ultrasons,* 24 Janvier 2002
- **PEI-JARN CHEN et al.** The measurements of ultrasound parameters on calcaneus by two-sided interrogation techniques. *Measurement Science and technology,* 2005, vol. 16
- *A novel Imaging System for Automatic Real-Time 3D patient-Specific Knee Model Reconstruction Using Ultrasound RF Data*